(19) Europäisches Patentamt
European Patent Office
Office européen des brevets

(11) Veröffentlichungsnummer: **0 281 092**
**A2**

(12) # EUROPÄISCHE PATENTANMELDUNG

(21) Anmeldenummer: **88103144.7**

(22) Anmeldetag: **02.03.88**

(51) Int. Cl.⁴: **C07D 501/46**, A61K 31/545

Patentansprüche für folgende Vertragsstaaten: ES & GR

(30) Priorität: **05.03.87 DE 3707019**

(43) Veröffentlichungstag der Anmeldung: **07.09.88 Patentblatt 88/36**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Lattrell, Rudolf, Dr.**
**Heuhohlweg 6h**
**D-6240 Königstein/Taunus(DE)**
Erfinder: **Dürckheimer, Walter, Dr.**
**Im Lerchenfeld 45**
**D-6234 Hattersheim am Main(DE)**
Erfinder: **Kirrstetter, Reiner, Dr.**
**Altenhainer Strasse 8a**
**D-6233 Kelkheim (Taunus)(DE)**
Erfinder: **Seibert, Gerhard, Prof. Dr.**
**Gläserweg 21**
**D-6100 Darmstadt(DE)**

(54) **Cephalosporinderivate und Verfahren zu ihrer Herstellung.**

(57) Cephalosporinderivate der allgemeinen Formel

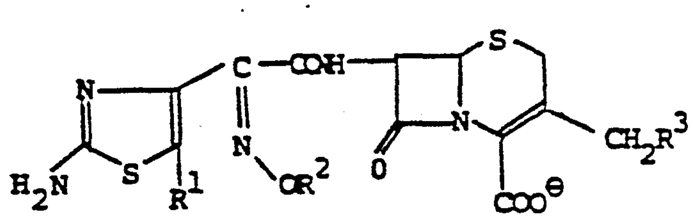

gegen bakterielle Infektionen wirksame pharmazeutische Präparate, die solche Cephemderivate enthalten, Verfahren zur Herstellung der Cephemderivate und der pharmazeutischen Präparate und Verwendung der Cephemderivate zur Bekämpfung bakterieller Infektionen.

EP 0 281 092 A2

## Cephalosporinderivate und Verfahren zu ihrer Herstellung

Die Erfindung betrifft neue Cephalosporinderivate, die in 3'-Stellung des Cephemrings durch einen über den Stickstoff gebundenden 5,6,7,8-Tetrahydroimidazo[1,2-a]pyridinium bzw. 5,6,7,8-Tetrahydroimidazo[1,5-a]pyridiniumrest substituiert sind. Sie besitzen eine sehr gute antimikrobielle Wirkung gegen grampositive und gramnegative Bakterien und sind deshalb als Arzneimittel zur Behandlung von mikrobiellen Infektionen gut geeignet. Die Erfindung betrifft weiterhin ein Verfahren zu ihrer Herstellung, sie enthaltende pharmazeutische Zubereitungen und deren Herstellung und die Verwendung zur Bekämpfung bakterieller Infektionen.

Gegenstand der Erfindung sind daher Cephalosporinderivate der allgemeinen Formel I

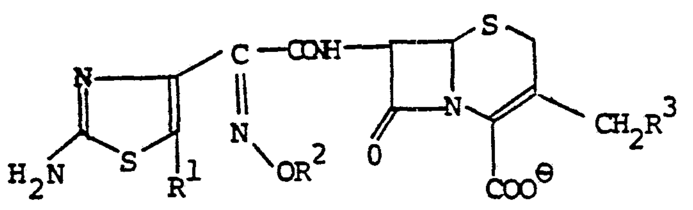

( I )

und deren physiologisch verträgliche Säureadditionssalze, worin die $R^2O$-Gruppe in syn-Stellung steht und die Substituenten $R^1$, $R^2$ und $R^3$ die folgenden Bedeutungen besitzen:

$R^1$ Wasserstoff oder Halogen

$R^2$ Wasserstoff,

$C_1$-$C_6$-Alkyl, das ein-oder mehrfach, gleich oder verschieden substituiert sein kann durch Halogen, Aryl, Heteroaryl, $C_1$-$C_4$-Alkylthio, $C_1$-$C_4$-Alkoxy, Nitril oder Carbamoyl, worin die Aminogruppe ein-oder zweifach substituiert sein kann durch $C_1$-$C_4$-Alkyl,

$C_2$-$C_6$-Alkenyl, das gegebenenfalls ein-oder mehrfach substituiert sein kann durch Halogen,

$C_2$-$C_6$-Alkinyl,

$C_3$-$C_7$-Cycloalkyl,

$C_4$-$C_7$-Cycloalkenyl,

$C_3$-$C_7$-Cycloalkylmethyl,

die Gruppe

$$-(CH_2)_n-(\overset{R^4}{\underset{R^5}{C}})_m-R^6,$$

worin m und n jeweils für 0 oder 1 steht und $R^4$ und $R^5$ gleich oder verschieden sein können und Wasserstoff oder eine $C_1$-$C_{11}$-Alkylgruppe bedeuten, oder zusammen mit dem Kohlenstoff, an das sie gebunden sind, eine Vinyliden-oder eine $C_3$-$C_7$-Cycloalkylidengruppe bilden,

$R^6$ eine Carboxy-oder $C_1$-$C_4$Alkyloxycarbonylgruppe,

$R^3$ einen 5,6,7,8-Tetrahydroimidazo[1,2-a]pyridiniumrest (a) oder einen 5,6,7,8-Tetrahydroimidazo[1,5-a]-pyridiniumrest (b)

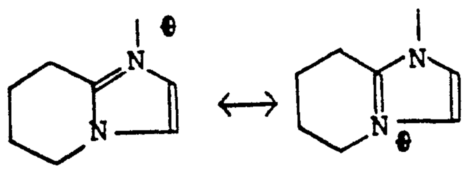
(a)

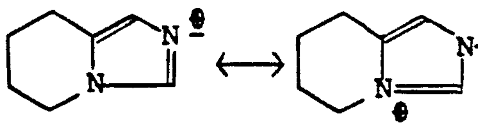
(b)

der ein-oder mehrfach, gleich oder verschieden substituiert sein kann durch $C_1$-$C_5$-Alkyl, das auch noch durch Hydroxy, $C_1$-$C_6$-Alkyloxy, Carboxy, $C_1$-$C_6$-Alkoxycarbonyl, Carbamoyl oder Amino substituiert sein kann;

$C_2$-$C_6$-Alkenyl, $C_3$-$C_6$Alkinyl, $C_3$-$C_6$-Cycloalkyl, $C_5$-$C_6$-Cycloalkenyl, $C_3$-$C_6$-Cycloalkylmethyl; Phenyl, Phenoxy oder Benzyl, die durch $C_1$-$C_4$-Alkyl, Hydroxy, $C_1$-$C_4$-Alkoxy substituiert sein können;

$C_1$-$C_6$-Alkoxy, $C_1$-$C_6$-Alkylthio, Sulfo, Trifluormethyl, Hydroxy, Mercapto, Amino, $C_1$-$C_6$-Alkylamino, Di-$C_1$-$C_6$-Alkylamino, Carboxy, $C_1$-$C_6$-Alkoxycarbonyl oder Carbamoyl, das am Stickstoff ein-oder zweimal substituiert sein kann durch $C_1$-$C_6$-Alkyl.

Als besonders bevorzugt kommen beispielsweise die folgenden Substituenten in Betracht:

$R^1$: Wasserstoff, Fluor, Chlor, Brom, insbesondere Wasserstoff und Chlor,

$R^2$: ein $C_1$-$C_4$-Alkylrest, wie z.B. Methyl, Ethyl, Propyl, Isopropyl, Butyl, insbesondere Methyl und Ethyl, der ein-oder mehrfach, insbesondere 1-bis 2-fach substituiert sein kann, insbesondere durch Fluor, wie z.B. Monofluormethyl oder Difluormethyl; durch Aryl, insbesondere durch Phenyl, wie z.B. Benzyl, durch 2-oder 3-oder 4-Tolyl, oder durch 2-oder 3-oder 4-Chlorphenyl; durch Heteroaryl, insbesondere durch 1,3-Thiazol-4-yl, wie z.B. 1,3-Thiazol-4-yl-methyl oder durch Imidazolyl, wie z.B. Imidazol-1-yl-ethyl; durch $C_1$-$C_4$-Alkylthio, insbesondere Methylthio, wie z.B. Methylthiomethyl; durch $C_1$-$C_4$-Alkyloxy, insbesondere Methyloxy und Ethyloxy, wie z.B. Methyloxymethyl, Ethyloxymethyl, Ethyloxyethyl; durch Nitril oder Carbamoyl, wobei die Aminogruppe auch noch ein-oder zweifach substituiert sein kann, beispielsweise durch $C_1$-$C_2$-Alkyl, wie z.B. N-Methyl-oder N-Ethyl-carbamoyl-methyl oder N,N-Dimethyl-carbamoyl-methyl,

ein $C_2$-$C_6$-vzw. ein $C_3$-$C_4$-Alkenylrest, wie z.B. 2-Propenyl, 2-Butenyl, der ein-oder mehrfach, vorzugsweise 1-bis 2-fach substituiert sein kann durch Halogen, insbesondere Chlor oder Brom, wie z.B. 3-Chlor-2-propenyl, 2-Brom-2-propenyl,

ein $C_3$-$C_6$-Alkinylrest, wie insbesondere Propargyl, ein $C_3$-$C_7$-Cycloalkylrest, wie insbesondere Cyclopropyl, Cyclobutyl, Cyclopentyl oder Cyclohexyl,

ein $C_3$-$C_7$-Cycloalkylmethylrest, wie insbesondere Cyclopropyl-und Cyclobutylmethyl,

ein $C_4$-$C_7$-Cycloalkenylrest, wie insbesondere Cyclopentenyl,

die Gruppe

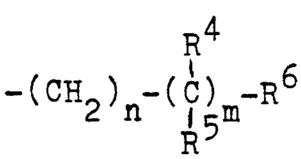

wobei $R^6$ die Gruppe COOH bedeutet und $R^4$ und $R^5$ gleich oder verschieden sein können und Wasserstoff, $C_1$-$C_4$-Alkyl, wie z.B. Methyl, Ethyl, Propyl, Isopropyl, Butyl sec. Butyl, vorzugsweise Methyl, Ethyl, insbesondere Methyl, bedeuten können oder

wobei $R^4$ und $R^5$ zusammen mit dem Kohlenstoffatom, an das sie gebunden sind, insbesondere eine Vinylidengruppe oder eine $C_3$-$C_7$-Cycloalkylidengruppe bilden können, wie z.B. Cyclopropyliden, Cyclobutyliden, Cyclopentyliden, Cyclohexyliden, vorzugsweise Cyclopropyliden, Cyclobutyliden oder Cyclopentyliden,

m = 0 oder 1,

n = 0 oder 1, wobei die Summe von m und n 1 oder 2 darstellt.

Bevorzugte Beispiele für die Gruppe

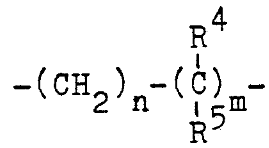

sind die folgenden:

Für den Fall, daß n = 0 und m = 1 ist:

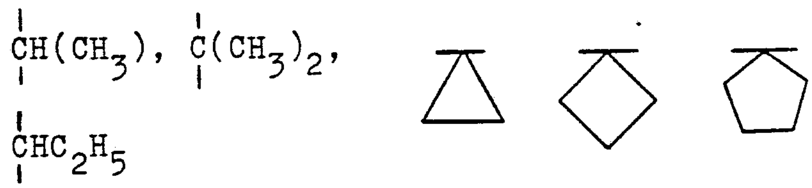

für den Fall, daß m = 0 und n = 1 ist:$-CH_2-$
und falls n und m = 1 sind: $-CH_2-C(=CH_2)-$.

Besonders bevorzugt stellt $R^3$ dar:
einen 5,6,7,8-Tetrahydroimidazo[1,2-a]pyridinium-oder einen 5,6,7,8-Tetrahydroimidazo[1,5-a]pyridiniumrest, der ein-oder mehrfach, insbesondere 1-bis 2-fach, vorzugsweise 1-fach substituiert sein kann, durch $C_1$-$C_3$-Alkyl, wie insbesondere Methyl, Ethyl, Propyl oder Isopropyl, vorzugsweise Methyl, also auch beispielsweise durch jeweils zwei Methyl-oder Ethylgruppen, wobei diese Alkylreste auch noch vorzugsweise 1-fach substituiert sein können. Als substituierte Alkylreste kommen insbesondere in Betracht
Hydroxy-$C_1$-$C_3$-alkyl, wie insbesondere Hydroxymethyl oder Hydroxyethyl,
$C_1$-$C_2$-Alkyloxy-$C_1$-$C_2$-alkyl, wie insbesondere Methyloxymethyl, Ethyloxymethyl, Methyloxyethyl oder Ethyloxyethyl, Carboxy-$C_1$-$C_4$-alkyl, wie insbesondere Carboxymethyl und Carboxyethyl;
$C_1$-$C_4$-Alkoxycarbonyl-$C_1$-$C_4$-alkyl, wie insbesondere Methyloxycarbonylmethyl, Ethyloxycarbonylmethyl, Methyloxycarbonylethyl;
Carbamoyl-$C_1$-$C_4$-alkyl, wie insbesondere Carbamoylmethyl oder Amino-$C_1$-$C_4$-alkyl, wie insbesondere Aminomethyl, Aminoethyl.

Für $R^3$ weiterhin besonders bevorzugt sind:
$C_3$-$C_4$-Alkenyl, wie insbesondere Allyl und Butenyl, wie z.B. 2-Methylallyl und Buten-3-yl,
$C_3$-Alkinyl, wie insbesondere Propargyl,
$C_3$-$C_6$-Cycloalkyl und $C_3$-$C_6$-Cycloalkyl-methyl, wobei sich die Kohlenstoffzahl auf den Cycloalkylteil bezieht, wie insbesondere Cyclopropyl, Cyclobutyl, Cyclopentyl und Cyclohexyl oder Cyclopropylmethyl, vorzugsweise Cyclopropyl und Cyclohexyl,
$C_5$-$C_6$-Cycloalkenyl, wie insbesondere Cyclopentenyl, Cyclohexenyl,
Phenyl, Phenoxy und Benzyl, die auch substituiert sein können, insbesondere durch Methyl, Ethyl, Hydroxy, Methoxy, Ethoxy wie z.B. Tolyl, Hydroxyphenyl, Methoxyphenyl, Ethoxyphenyl;
$C_1$-$C_4$-Alkoxy, wie insbesondere Methoxy, Ethoxy, Propoxy, Isopropoxy, Butoxy, Isobutoxy und tert.-Butoxy, vorzugsweise Methoxy;
$C_1$-$C_4$-Alkylthio, wie insbesondere Methylthio, Ethylthio, Propylthio und Isopropylthio;
Sulfo, Trifluormethyl, Hydroxy, Mercapto, Carboxy, wobei Carboxy und Hydroxy bevorzugt sind,
Amino, $C_1$-$C_4$-Alkylamino, wie insbesondere Methylamino, Ethylamino, Di-$C_1$-$C_4$-Alkylamino, wie insbesondere Dimethylamino, Methylethylamino,
$C_1$-$C_4$-Alkoxycarbonyl, wie insbesondere Methoxycarbonyl, Carbamoyl, das am Stickstoffatom einmal oder zweimal substituiert sein kann durch $C_1$-$C_4$-Alkyl, wie insbesondere N-Methyl-, N-Ethyl, N,N-Dimethylcarbamoyl, vorzugsweise das unsubstituierte Carbamoyl.

Gegenstand der Erfindung ist weiterhin ein Verfahren zur Herstellung von Verbindungen der Formel I und ihrer physiologisch verträglichen Säureadditionssalze, das dadurch gekennzeichnet ist, daß man

a) eine Verbindung der allgemeinen Formel II

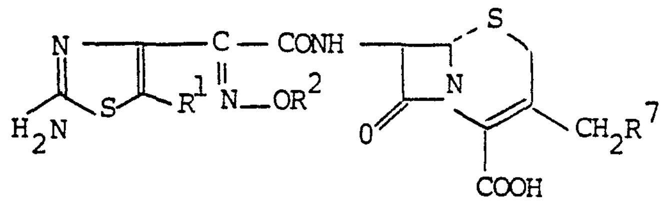

(II)

oder deren Salze, worin $R^1$ und $R^2$ die in Formel I genannte Bedeutung haben, die Aminogruppe auch geschützt sein kann, und $R^7$ eine durch Tetrahydroimidazopyridin oder dasjenige Tetrahydroimidazopyridin-Derivat, das dem Rest $R^3$ der Formel I entspricht, austauschbare Gruppe bedeutet, mit Tetrahydroimidazopyridin oder diesem Tetrahydroimidazopyridinderivat umsetzt,

α) eine gegebenenfalls vorhandene Schutzgruppe abspaltet und

β) falls erforderlich, das erhaltene Produkt in ein physiologisch verträgliches Säureadditionssalz überführt,

oder

b) eine Verbindung der allgemeinen Formel III

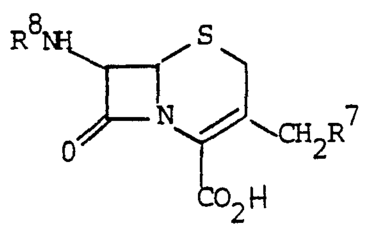

(III)

worin $R^7$ die vorstehend für die Formel II genannte Bedeutung hat und $R^8$ für Wasserstoff oder eine Aminoschutzgruppe steht, mit Tetrahydroimidazopyridin oder dem Tetrahydroimidazopyridin-derivat, das dem in Formel I definierten Rest $R^3$ zugrunde liegt, umsetzt unter Bildung der Verbindung der allgemeinen Formel IV,

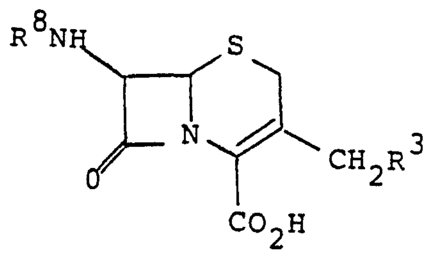

(IV)

in der $R^3$ und $R^8$ die oben genannte Bedeutung haben und

α) eine gegebenenfalls vorhandene Aminoschutzgruppe abspaltet und

β) die Verbindung IV, worin $R^8$ Wasserstoff bedeutet, entweder als solche oder in Form eines reaktionsfähigen Derivates mit einer 2-syn-Oxyiminoessigsäure der allgemeinen Formel V

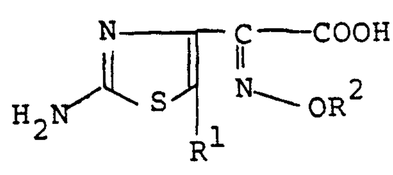

(V)

worin $R^1$ und $R^2$ die genannte Bedeutung besitzen und die Aminogruppe auch in geschützter Form vorliegen kann, oder mit einem an der Carboxylgruppe aktivierten Derivat dieser Verbindung umsetzt und

α) eine gegebenenfalls vorhandene Schutzgruppe abspaltet und

β) falls erforderlich, das erhaltene Produkt der allgemeinen Formel I in ein physiologisch verträgliches Säureadditionssalz überführt.

Soll die Herstellung der Verbindung der allgemeinen Formel I nach der **Verfahrensvariante a)** durch nucleophilen Austausch von $R^7$ in den Verbindungen der allgemeinen Formel II durch Tetrahydroimidazopyridin oder eines der angegebenen Tetrahydroimidazopyridinderivate erfolgen. so kommen als Reste $R^7$ insbesondere in Betracht Acyloxyreste mit niederen aliphatischen Carbonsäuren, vorzugsweise mit 1 bis 4 C-Atomen, wie z.B.: Acetoxy oder Propionyloxy, insbesondere Acetoxy, die gegebenenfalls substituiert sein können, wie z.B.: Chloracetoxy oder Acetylacetoxy. Für $R^7$ kommen auch andere Gruppen in Betracht, wie beispielsweise Carbamoyloxy oder ein Halogenatom, wie z.B. Chlor, Brom oder Jod.

Erfindungsgemäß werden bei der nucleophilen Austauschreaktion Ausgangsverbindungen der allgemeinen Formel II, in denen $R^7$ die vorgenannte Bedeutung hat, eingesetzt, oder deren Salze, wie z.B. ein Natrium-oder Kaliumsalz. Die Reaktion wird in einem Lösungsmittel, vorzugsweise in Wasser Formamid oder in einem Gemisch aus Wasser und einem mit Wasser leicht mischbaren organischen Lösungsmittel, wie z.B. Aceton, Dioxan, Acetonitril, Dimethylformamid, Dimethylsulfoxid oder Äthanol durchgeführt. Die Reaktionstemperatur liegt im allgemeinen im Bereich von etwa 10 bis etwa 100°C, vorzugsweise zwischen 20 und 80°C. Die Tetrahydroimidazopyridinkomponente wird in Mengen zugegeben, die zwischen etwa äquimolaren Mengen und einem bis zu etwa 5-fachen Überschuß liegen. Der Austausch des Restes $R^7$ wird durch Anwesenheit von Neu tralsalzionen, vorzugsweise von Jodid-oder Thiocyanationen, im Reaktionsmedium erleichtert. Insbesondere werden etwa 10 bis etwa 30 Äquivalente Kaliumjodid, Natriumjodid, Kaliumthiocyanat oder Natriumthiocyanat zugegeben. Die Reaktion wird vorteilhaft in der Nähe des Neutralpunktes, vorzugsweise bei einem pH-Wert im Bereich von etwa 5 bis etwa 8 durchgeführt.

Die nucleophile Austauschreaktion an Verbindungen der allgemeinen Formel II, kann auch so erfolgen, daß die Reaktion in Gegenwart von Tetrahydroimidazopyridin oder der den Resten $R^3$ entsprechenden Tetrahydroimidazopyridinderivate und von Tri-$C_1$-$C_4$-alkyljodsilanen, wie z.B. Trimethyl-oder Triäthyljodsilan, vorgenommen wird. Dabei kann so verfahren werden, daß die Verbindung II wobei $R^7$ beispielsweise für Acetoxy steht, zuerst mit Trimethyljodsilan nach den im folgenden genannten Reaktionsbedingungen zur Reaktion gebracht wird, die gebildete Verbindung II mit $R^7$ = J isoliert und anschließend mit Tetrahydroimidazopyridin oder dem Tetrahydroimidazopyridinderivat umgesetzt wird, oder die 3-$CH_2$J-Verbindung in situ durch Zugabe des Tetrahydroimidazopyridins bzw. seines Derivates zur Reaktion gebracht wird. Statt Trimethyljodsilan kann beispielsweise auch eine Reaktionsmischung aus Jod und Hexamethyldisilan, die vorher bei Temperaturen zwischen etwa 60 und 120°C in literaturbekannter Weise zur Reaktion gebracht wurden, wobei Trimethyljodsilan entsteht, verwendet werden. Statt Trimethyljodsilan kann mit demselben guten Ergebnis auch Triethyljodsilan, das in literaturbekannter Weise hergestellt wird, verwendet werden. Die Reaktion wird ausgeführt bei Temperaturen zwischen etwa -5° und +100°C, vorzugsweise zwischen +10°C und +80°C.

Geeignete inerte aprotische Lösungsmittel sind z.B. chlorierte Kohlenwasserstoffe, wie Methylenchlorid, Chloroform, Dichlorethan, Trichlorethan, Tetrachlorkohlenstoff oder Niederalkylnitrile, wie Acetonitril oder Propionitril oder Frigene; insbesondere ist Methylenchlorid ein hervorragendes Lösungsmittel.

Das dem Rest $R^3$ entsprechende Tetrahydroimidazopyridin bzw. dessen Derivat wird in mindestens stöchiometrischer Menge bis zu einem etwa zwanzigfachen Überschuß zugegeben. Vorzugsweise wird mit solchen Mengen gearbeitet, daß die freiwerdende Jodwasserstoffmenge gebunden wird und noch mindestens 1 Mol, vorzugsweise 1,5-5 Mol Tetrahydroimidazopyridin bzw. dessen Derivat für die Substitution zur Verfügung stehen.

Da neben der auszutauschenden Gruppe $R^7$ in der Ausgangsverbindung II auch andere funktionelle Gruppen, wie z.B. die Aminogruppe oder die Carboxylgruppe, mit Trimethyljodsilan reagieren, wird letzteres in mindestens doppeltem bis zu etwa fünfzehnfachen, vorzugsweise in drei-bis zehnfachem Überschuß zugegeben.

Derartige funktionelle Gruppen können auch durch Zugabe eines Silylierungsmittels wie z.B. Bistrimethylsilylacetamid, N-Methyl-N-trimethylsilyltrifluoracetamid; Bistrimethylsilyltrifluoracetamid, Trimethylchlorsilan, Hexamethyldisilazan, Bistrimethylsilylharnstoff, vorsilyliert werden, entweder ohne oder in Gegenwart einer Base, vorzugsweise des gewünschten, der Gruppe $R^3$ zugrundeliegenden Tetrahydroimidazopyridins bzw. dessen Derivates in den vorstehend beschrieben Mengen. Anschließend wird Trimethyljodsilan in mindestens stöchiometrischer Menge oder auch im Überschuß, vorzugsweise in einem doppelten bis zu einem zehnfachen Überschuß zugegeben.

Liegt die Aminogruppe in den Verbindungen der Formel II und V in geschützter Form vor, so eignen sich als Aminoschutzgruppe, z.B. gegebenenfalls substituiertes Alkyl, wie beispielsweise tert.-Butyl, tert.-Amyl; Benzyl, p-Methoxybenzyl, Trityl, Benzhydryl, vorzugsweise Trityl; Trialkylsilyl, wie beispielsweise Trimethylsilyl; gegebenenfalls substituiertes aliphatisches Acyl, wie z.B. Formyl, Chloracetyl, Bromacetyl, Trichloracetyl und Trifluoracetyl, vorzugsweise Formyl; oder gegebenenfalls substituiertes Alkoxycarbonyl, wie beispielsweise Trichloräthoxycarbonyl, Benzyloxycarbonyl oder tert.-Butoxycarbonyl, vorzugsweise tert.-Butyloxycarbonyl und Benzyloxycarbonyl; oder Dimethylaminomethylen.

Die Schutzgruppe kann nach der Austauschreaktion in an sich bekannter Weise abgespalten werden,

z.B. die Tritylgruppe mittels einer Carbonsäure, wie z.B. Essigsäure, Trifluoressigsäure, Ameisensäure, oder die Benzyloxycarbonylgruppe hydrogenolytisch.

Die Reaktionsprodukte der Formel I können beispielsweise nach Zugabe von Wasser oder wäßrigen Mineralsäuren, z.B. verdünnter HCl, HBr, HJ oder $H_2SO_4$, aus der wäßrigen Phase in üblicher Weise, z.B. durch Gefriertrocknen der Wasserphase, Chromatographie oder Fällung durch Zugabe von organischen Lösungsmitteln, isoliert werden. Vorzugsweise werden die Reaktionsprodukte durch Ausfällen aus der Reaktionslösung in Form eines schwerlöslichen Salzes, beispielsweise eines Hydrojodidsalzes, isoliert.

Für den Fall, daß $R^7$ für eine Carbamoyloxygruppe steht, wird die Austauschreaktion analog durchgeführt. Steht $R^7$ für Halogen, insbesondere Brom oder Jod, so erfolgt der Austausch in literaturbekannter Weise.

Liegt die Verbindung II in Form eines reaktionsfähigen Derivates vor, so kommen beispielsweise Silylderivate in Betracht, die bei der Umsetzung von Verbindungen der allgemeinen Formel II mit einer Silylverbindung gebildet werden, wie z.B. Trimethylchlorsilan oder Bis(trimethylsilyl)acetamid. In diesem Fall wird die Umsetzung zweckmäßig in Gegenwart eines Lösungsmittel, wie Methylenchlorid, Dimethylformamid oder Acetonitril, vorgenommen.

Nach der **Verfahrensvariante b)** werden die Verbindungen der allgemeinen Formel I durch Acylierung von Verbindungen der allgemeinen Formel IV oder deren Additionssalze, beispielsweise mit Chlorwasserstoffsäure, Bromwasserstoffsäure, Salpetersäure, Schwefelsäure, Phosphorsäure oder einer organischen Säure, wie z.B. Methansulfonsäure oder Toluolsulfonsäure, mit Carbonsäuren der allgemeinen Formel V oder mit einem reaktionsfähigen Derivat einer solchen Säure erhalten.

Es ist dabei nicht erforderlich, die Verbindungen der allgemeinen Formel IV zu isolieren. Die Reaktion kann auch so durchgeführt werden, daß man Verbindungen der allgemeinen Formel III, beispielsweise 7-Aminocephalosporansäure oder 3-Jodmethyl-7-amino-ceph-3-em-4-carbonsäure oder deren reaktionsfähige Derivate in einem geeigneten Lösungsmittel mit dem dem Rest $R^3$ in der allgemeinen Formel IV entsprechenden Tetrahydroimidazopyridin bzw. dessen Derivat umsetzt und die entstandene Verbindung der allgemeinen Formel IV in situ zu den Verbindungen der allgemeinen Formel I acyliert. Erfindungsgemäß werden bei dieser Reaktion Ausgangsverbindungen der allgemeinen Formel III, in denen $R^7$ für Jod steht, eingesetzt. Geeignete Lösungsmittel sind chlorierte Kohlenwasserstoffe, wie z.B. Methylenchlorid und Chloroform; Ether, wie z.B. Diethylether, Tetrahydrofuran, Dioxan, Acetonitril und Amide, wie vorzugsweise Dimethylformamid und Dimethylacetamid. Es kann sich auch als vorteilhaft erweisen, Gemische der genannten Lösungsmittel zu verwenden.

Liegt in den Verbindungen der allgemeinen Formel III ein reaktionsfähiges Derivat vor, so kommen insbesondere Silylderivate in Betracht, die bei der Umsetzung von Verbindungen der allgemeinen Formel III mit Silylverbindungen, wie z.B. Trimethylchlorsilan, Bis(trimethylsilyl)trifluoracetamid usw. gebildet werden. Die dem Rest $R^3$ entsprechende Base wird in mindestens stöchiometrischer Menge bis zu einem etwa zehnfachen Überschuß, vorzugsweise 1,5 bis 5 Äquivalenten verwendet. Die Reaktion wird bei Temperaturen zwischen etwa -50 und +100°C, vorzugsweise zwischen +20 und +50°C ausgeführt.

Die Verbindungen der allgemeinen Formel IV können auch aus Verbindungen der allgemeinen Formel III wobei $R^7$ z.B. für Acetoxy steht, in analoger Weise wie vorstehend für die Verbindungen der allgemeinen Formel II beschrieben, dargestellt werden.

Werden die Carbonsäure der allgemeinen Formel V sowie ihre an der Aminogruppe geschützten Derivate selbst als Acylierungsmittel eingesetzt, so wird zweckmäßig in Gegenwart eines Kondensationsmittels, beispielswise eines Carbodiimids, wie beispielsweise N,N'-Dicyclohexylcarbodiimid, gearbeitet.

Die Aktivierung von Carbonsäuren der allgemeinen Formel V kann in in besonders günstiger Weise durch Behandlung mit bestimmten Carbonsäureamiden und beispielsweise Phosgen, Phosphorpentachlorid, Tosylchlorid, Thionylchlorid oder Oxalylchlorid erfolgen, wie sie z.B. in der deutschen Patentschrift 28 04 040 beschrieben wird.

Als aktivierte Derivate der Carbonsäuren der allgemeinen Formel V eignen sich insbesondere auch Halogenide, vorzugsweise Chloride, die in an sich bekannter Weise durch Behandlung mit Halogenierungsmitteln, wie z.B. Phosphorpentachlorid, Phosgen oder Thionylchlorid unter für die Cephalosporinchemie literaturbekannten, schonenden Reaktionsbedingungen erhalten werden.

Als aktivierende Derivate der Carbonsäuren der allgemeinen Formel V eignen sich ferner die Anhydride und gemischten Anhydride, Azide, aktivierten Ester und Thioester. Als gemischte Anhydride sind besonders geeignet solche mit niederen Alkansäuren, wie z.B. Essigsäure und besonders bevorzugt solche mit substituierten Essig säuren, wie z.B. Trichloressigsäure, Pivalinsäure und Cyanessigsäure. Besonders geeignet sind aber auch die gemischten Anhydride mit Kohlensäurehalbestern, die man beispielsweise durch Umsetzung der Carbonsäuren der Formel V, in denen die Aminogruppe geschützt ist, mit Chlorameisensäurebenzylester, -p-nitrobenzylester, -isobutylester, -ethylester oder -allylester gewinnt.

Als aktivierte Ester eignen sich vorzugsweise diejenigen mit p-Nitrophenol, 2,4-Dinitrophenol, Methylcyanhydrin, N-Hydroxysuccinimid und N-Hydroxyphthalamid, insbesondere diejenigen mit 1-Hydroxybenzotriazol und 6-Chlor-1-hydroxybenzotriazol. Besonders bevorzugte Thioester sind beispielsweise diejenigen mit 2-Mercaptobenzothiazol und 2-Mercaptopyridin. Die aktivierten Derivate können als isolierte Substanzen aber auch in situ umgesetzt werden.

Im allgemeinen erfolgt die Umsetzung der Cephemderivate der allgemeinen Formel IV mit einer Carbonsäure der allgemeinen Formel V oder einem aktivierten Derivat derselben in Gegenwart eines inerten Lösungsmittels. Insbesondere eignen sich chlorierte Kohlenwasserstoffe, wie vorzugsweise Methylenchlorid und Chloroform; Äther, wie z.B. Diäthyläther, vorzugsweise Tetrahydrofuran und Dioxan; Ketone, wie vorzugsweise Aceton und Butanon; Amide, wie vorzugsweise Dimethylformamid und Dimethylacetamid, oder Pyridin. Es kann sich auch als vorteilhaft erweisen, Gemische der genannten Lösungsmittel zu verwenden. Dies ist oftmals dann der Fall, wenn die Cephemverbindung der allgemeinen Formel IV mit einem in situ erzeugten aktivierten Derivat einer Carbonsäure der Formel V umgesetzt wird.

Die Umsetzung von Cephemverbindungen der Formel IV mit Carbonsäuren der Formel V bzw. deren aktivierten Derivaten kann in einem Temperaturbereich von etwa -80 bis etwa +80°C, vorzugsweise zwischen etwa -20°C und Raumtemperatur erfolgen.

Die Reaktionsdauer hängt von den Reaktanten, der Temperatur und dem Lösungsmittel bzw. dem Lösungsmittelgemisch ab und liegt normalerweise zwischen 1/4 und etwa 72 Stunden.

Die Reaktion mit Säurehalogeniden kann gegebenenfalls in Gegenwart eines säurebindenen Mittels zur Bindung des freigesetzten Halogenwasserstoffs durchgeführt werden. Als solche eignen sich insbesondere tertiäre Amine, wie z.B. Triäthylamin oder Dimethylanilin, anorganische Basen, wie z.B. Kaliumcarbonat oder Natriumcarbonat, Alkylenoxide, wie z.B, Propylenoxid. Auch die Anwesenheit eines Katalysators, wie z.B. von Dimethylaminopyridin, kann gegebenenfalls von Vorteil sein. Liegt in den Verbindungen der allgemeinen Formel IV die Aminogruppe in Form eines reaktionsfähigen Derivats vor , so kann es sich um ein solches handeln, wie aus der Literatur für Amidierungen bekannt ist. So kommen beispielsweise Silylderivate in Betracht, die bei der Umsetzung von Verbindungen der allgemeinen Formel IV mit einer Silylverbindung gebildet werden, wie z.B. Trimethylchlorsilan oder Bis(trimethylsilyl)acetamid. Wird die Umsetzung mit einer solchen, an der Aminogruppe aktivierten Verbindung durchgeführt, so ist es zweckmäßig, die Reaktion in einem inerten Lösungsmittel, wie z.B. Methylenchlorid, Tetrahydrofuran oder Dimethylformamid durchzuführen.

Als physiologisch verträgliche Säureadditionssalze der Verbindungen der allgemeinen Formel I seien beispielsweise erwähnt solche mit Chlorwasserstoffsäure, Bromwasserstoffsäure, Salpetersäure, Phosphorsäure, Schwefelsäure oder organische Säuren, wie z.B. Methansulfonsäure, p-Toluolsulfonsäure oder Maleinsäure.

Die erfindungsgemäß eingesetzten Tetrahydroimidazopyridine und andere Ausgangsverbindungen sind literaturbekannt und können nach literaturbekannten Verfahren erhalten werden.

Die erfindungsgemäß erhaltenen Verbindungen der allgemeinen Formel I und ihre physiologisch verträglichen Säureadditionssalze zeigen bemerkenswert gute antibakterielle Wirksamkeit sowohl gegen grampositive als auch gramnegative bakterielle Keime.

Auch gegen penicillinase-und cephalosporinase-bildende Bakterien sind die Verbindugnen der Formel I unerwartet gut wirksam. Da sie zudem günstige toxikologische und pharmakologische Eigenschaften zeigen, stellen sie wertvolle Chemotherapeutika dar.

Die Erfindung betrifft somit auch Arznei-Präparate zur Behandlung von mikrobiellen Infektionen, die durch einen Gehalt an einer oder mehreren oder erfindungsgemäßen Verbindungen charakterisiert sind.

Die erfindungsgemäßen Produkte können auch in Kombinationen mit anderen Wirkstoffen, beispielsweise aus der Reihe der Penicilline, Cephalosporine oder Aminoglykoside zur Anwendung kommen.

Die Verbindungen der allgemeinen Formel I und ihre physiologischen verträglichen Säureadditionssalze können oral, intramuskulär oder intravenös verabfolgt werden.

Arzneipräparate, die eine oder mehrere Verbindungen der allgemeinen Formel I als Wirkstoff enthalten, können hergestellt werden, indem man die Verbindungen der Formmel I mit mehreren pharmakologisch verträglichen Trägerstoffen oder Verdünnungsmittel, wie z.B. Füllstoffen, Emulgatoren, Gleitstoffen, Geschmackskorrigentien, Farbstoffen oder Puffersubstanzen vermischt in eine geeignete galenische Zubereitungsform bringt, wie beispielsweise Tabletten, Dragees, Kapseln oder eine zur parenteralen Applikation geeignete Suspension oder Lösung.

Als Träger-oder Verdünnungsmittel seien beispielsweise erwähnt Traganth, Milchzucker, Talkum, Agar-Agar, Polyglykole, Äthanol und Wasser. Puffersubstanzen sind beispielsweise organische Verbindungen, wie z.B. N,N'-Dibenzyläthylendiamin, Diäthanolamin, Äthylendiamin, N-Methylglucamin, N-Benzylphenäthylamin, Diäthylamin, Tris(hydroxymethyl)aminomethan, oder anorganische Verbindungen wie z.B.,

Phosphatpuffer, Natriumbicarbonat, Natriumcarbonat. Für die parenterale Applikation kommen vorzugsweise Suspensionen oder Lösungen in Wasser mit oder ohne Puffersubstanzen in Betracht. Es ist auch möglich, die Wirkstoffe als solche ohne Träger-oder Verdünnungsmittel in geeigneter Form, beispielsweise in Kapseln, zu applizieren.

geeignete Dosen der Verbindungen der allgemeinen Formel I oder ihrer physiologisch verträglichen Säureadditionssalze liegen bei etwa 0,4 bis 20 g/Tag, vorzugsweise bei 0,5 bis 4 g Tag, für einen Erwachsenen von etwa 60 kg Körpergewicht.

Es können Einzel-oder im allgemeinen Mehrfachdosen verabreicht werden, wobei die Einzeldosis den Wirkstoff in einer Menge von etwa 50 bis 1000 mg, vorzugsweise von etwa 100 bis 500 mg, enthalten kann.

Die folgenden Ausführungsbeispiele für erfindungsgemäßhergestellbare syn-Verbindungen dienen zur weiteren Erläuterung der Erfindung, schränken sie jedoch nicht darauf ein.

**Beispiel 1:**

7-[2-(2-Aminothiazol-4-yl)-2-(Z)-methoxyiminoacetamido]-3-[(5,6,7,8-tetrahydroimidazo[1,5-a]pyridino-2-yl)-methyl] -ceph-3-em-4-carboxylat

**Verfahren a) Variante 1**

Eine Mischung von 0,91 g (2 mmol) 7-[2-(2-Aminothiazol)-2-syn-methoxyiminoacetamido]-cephalosporansäure, 1,85 g (7,2 mmol) Bis(trimethylsilyl)trifluoroacetamid (BSTFA) und 5 ml Methylendichlorid wird 1 Stunde unter Rückfluß erhitzt. Die Lösung wird auf 20°C gekühlt, 1,04 g (5,2 mmol) Trimethyljodsilan zugegeben und weitere 20 Min bei Raumtemperatur gerührt. Es wird im Vakuum eingeengt, der ölige Rückstand in 4 ml Acetonitril gelöst und eine Lösung von 305 mg (2,5 mmol) 5,6,7,8-Tetrahydroimidazo [1,5-a]pyridin und 1,23 g (4,8 mmol) BSTFA in 2 ml Acetonitril zugegeben. Die dunkelgefärbte Lösung wird 4 Stunden bei Raumtemperatur belassen. Es werden 0,3 ml Wasser zugegeben und der gebildete Niederschlag abgesaugt, mit Aethanol und Aceton gewaschen und getrocknet. Dieses rohe Hydrojodidsalz der Titelverbindung (Ausbeute 1,0 g) wird in wässriger Natriumbicarbonatlösung gelöst und über eine "Lobar B"-Kieselgelsäule (Fa. Merck, Darmstadt, FRG) mit Aceton-Wasser (3:1) chromatographiert. Nach Gefriertrocknung der Produktfraktionen erhält man 175 mg (17 %) der Titelverbindung als gelblichen amorphen Feststoff.

$^1$H-NMR (270 MHz, DMSO-$d_6$): $\delta$ = 1,7 - 1,83 (2H, m, $CH_2$); 1,83 - 1,96 (2H, m, $CH_2$); 2,72 - 2,82 (2H, m, $CH_2$); 3,06 und 3,50 (2H, AB, J=18Hz, $SCH_2$); 3,80 (3H, s, $OCH_3$); 4,12 -4,22 (2H, m, $CH_2$); 4,66 und 5,14 (2H, AB, J=15Hz, $CH_2N^{\oplus}$) 5,00 (1H, d, J=5Hz, 6-H); 5,59 (1H, dd, J=5 und 8Hz, 7-H); 6,71 (1H, s, Thiazol); 7,12 (2H, s, $NH_2$); 7,78 (1H, s, Im-H); 9,14 (1H, s, Im-H); 9,47 (1H, d, J=8Hz, NH).

**Verfahren a), Variante 2**

Ein Gemisch aus 228 mg (0,5 mmol) 7-[2-(2-Aminothiazol)-2-syn-methoxyiminoacetamido]-cephalosporansäure, 3,32 g (20 mmol) Kaliumjodid, 610 mg (5 mmol) 5,6,7,8-Tetrahydroimidazo[1,5-a]-pyridin, 3 ml Wasser und 1 ml Aceton wird 3 Stunden bei 68°C unter Rühren erhitzt. Nach dem Erkalten wird die Lösung über Kieselgel (Lobar B-Säule, Merck) mit Aceton-Wasser (3:1) chromatographiert. Die Produktfraktionen werden eingeengt und gefriergetrocknet. Man erhält 65 mg (25 %) eines amorphen Feststoffs, der in allen Eigenschaften mit dem vorstehend beschriebenen identisch ist.

**Verfahren b), Variante 1**

0,2 g (1 mmol) 2-(2-Aminothiazol-4-yl)-2-syn-methoxyiminoessigsäure werden in 3 ml N,N-Dimethylformamid (DMF) gelöst. Nach Zugabe von 0,14 g (1,05 mmol) 1-Hydroxybenztriazolhydrat und 0,21 g (1 mmol) N,N'-Dicyclohexylcarbodiimid wird 3 Stunden bei Raumtemperatur gerührt. Vom Dicyclohexylharnstoff wird filtriert und zum Filtrat eine Lösung von 268 mg (0,8 mmol) 7-Amino-3-[(5,6,7,8-tetrahydroimidazo-[1,5-a]pyridinio-2-yl)methyl]ceph-3-em-4-carb oxylat in 4 ml DMF und 0,5 ml Wasser zugegeben. Nach 24 Stunden bei Raumtemperatur wird die Mischung im Vakuum eingeengt und der Rückstand über Kieselgel (Lobar B, Merck) mit Aceton-Wasser (3:1) chromatographiert. Ausbeute 206 mg (50 %) farbloser Feststoff. Er ist mit dem nach Verfahren a) erhaltenen Produkt identisch.

**Verfahren b), Variante 2**

Zu einer Lösung von 339 mg (1 mmol) 7-Amino-3-jodmethylceph-3-em-4-carbonsäure und 0,73 ml (3 mmol) Bistrimethylsilylacetamid in 10 ml DMF werden 244 mg (2 mmol) 5,6,7,8-Tetrahydroimidazo[1,5-a]-pyridin gegeben und die Lösung 2 Stunden bei Raumtemperatur belassen. Sodann werden 0,5 ml Wasser und 319 mg (1 mmol) 2-(2-Aminothiazol-4-yl)-2-syn-methoxyiminoessigsäure-1-hydroxybenztriazol-Aktivester zugegeben und weitere 17 Stunden bei Raumtemperatur gerührt. Die Lösung wird im Vakuum eingeengt und der ölige Rückstand über Kieselgel (Lobar B, Merck) mit Aceton-Wasser (3:1) chromatographiert. Gefriertrocknung der Produktfraktionen liefern 170 mg (33 %) eines amorphen Feststoffs, der in allen Eigenschaften mit dem nach Verfahren a) erhaltenen Produkt identisch ist.

Analog Beispiel 1, Verfahren a) oder b) werden die nachfolgend aufgeführten Beispiele als amorphe Feststoffe erhalten,die der allgemeinen Formel I a entsprechen und worin $R^1$, $R^2$ und $R^3$ die in der Tabelle 1 angegebene Bedeutung haben.

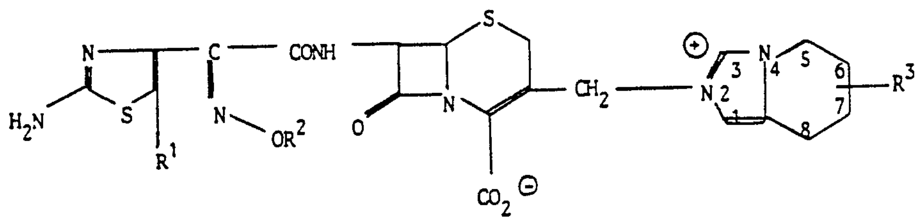

(Ia)

Tabelle 1

| Beispiel | $R^1$ | $R^2$ | $R^{3'}$ | $^1$H-NMR (DMSO-$d_6$), 60 MHz: δ (ppm)= |
|---|---|---|---|---|
| 2 | H | $C_2H_5$ | H | 270MHz:1,21 (3H, t, $CH_2CH_3$), 1,7 - 2,0 (4H, m, 2 x $CH_2$); 2,7 - 2,85 (2H, m, $CH_2$); 3,08 und 3,49 (2H, AB, J=18Hz, $SCH_2$); 4,07 (2H, q, $CH_2CH_3$), 4,1 - 4,22 (2H, m, $CH_2$); 4,68 und 5,13 (2H, AB, J=14Hz, $CH_2N^{\oplus}$); 5,02 (1H, d, J=5Hz, 6-H); 5,62 (1H, dd, J=5 und 8Hz, 7-H), 6,70 (1H, s, Thiazol); 7,20 (bs, $NH_2$); 7,78 und 9,14 (je 1H, s, Im-H); 9,43 (1H, d, J=8Hz, NH) |
| 3 | H | $CHF_2$ | H | 1,7 - 2,0 (4H, m, 2 x $CH_2$); 2,7 - 2,8 (2H, m, $CH_2$); 3,1 - 3,6 (2H, AB, $SCH_2$); 3,9 - 4,2 (2H, m, $CH_2$); 4,4 - 5,2 (2H, AB, $CH_2N^{\oplus}$); 5,04 (1H, d, J=5Hz, 6-H); 5,62 (1H, dd, J=5 und 8Hz, |

| Beispiel | $R^1$ | $R^2$ | $R^{3'}$ | $^1H$-NMR(DMSO-$d_6$), 60MHz: δ (ppm)= |
|---|---|---|---|---|
| | | | | (1H, t, J=70Hz, $CHF_2$); 6,72 (1H, s, Thiazol); 7,15 (2H, s, $NH_2$); 7,76 und 9,06 (je 1H, s, Im-H); 9,35 (1H, d, J=8Hz, NH); |
| 4 | H | $CH_2CH=CH_2$ | H | 1,7-2,0 (4H, m, 2x$CH_2$), 2,7-2,85 (2H,m,$\underline{CH_2}$), 3,0-3,5 (2H, AB, $SCH_2$); 4,1-4,2 (2H,m,$CH_2$), 4,55 - 4,7 (2H,m,$OCH_2$), 5,02 (1H, d, J=5Hz, 6-H), 4,6-6,0 (5H, m, $CH=CH_2$ und $CH_2N^{\oplus}$) 5,60 (1 H,dd, J=5 und 8 Hz, 7-H), 6,73 (1 H, s, Thiazol), 7,15 (2H, s, $NH_2$), 7,75 und 9,03 (je 1 H,s,Im-H); 9,28 (1H, d, J=8Hz, NH) |
| 5 | H | $CH_2C{\equiv}CH$ | H | 1,65-2,05 (4H, m, 2x$CH_2$), 2,6 - 2,85 (3H, m, $CH_2$ und CH), 3,03 und 3,42 ( 2H, AB ,J = 18Hz, $SCH_2$),4,1 - 4,25 (2H,m,$CH_2$) 4,51 2H, bs, $OCH_2$), 4,71 und 5,16 (2H, AB, J = 15Hz, $CH_2N^{\oplus}$); 5,02 (1H, d, J=5Hz, 6-H); 5,60 (1H, dd,J= 5 und 8Hz, 7-H), 6,72 (1H, s, Thiazol), 7,15 (2H, s, $NH_2$), 7,76 und 9,08 (je 1H, s, Im-H), 9,30 (1H, d, J=8Hz, NH) |
| 6 | H | $CH_2CONH_2$ | H | 1,7-2,15 (4H, m, 2 x $CH_2$), 2,6-2,7 (2H, m, $CH_2$), 3,1 - 3,4 (2H, AB,$SCH_2$), 4,1 - 4,22 (2H, m, $CH_2$), 4,42 (2H, s, $OCH_2$), 4,7 - 5,1 (2H, AB, $CH_2N^{\oplus}$); 5,02 (1H, d, J=5Hz, 6-H); 5,68 (1H, dd, |

| Beispiel | $R^1$ | $R^2$ | $R^{3'}$ | $^1H$-NMR(DMSO-$d_6$), 60MHz: $\delta$ (ppm)= |
|---|---|---|---|---|
| | | | | J=5 und 8Hz, 7-H); 6,76 (1H, s, Thiazol); 7,10 und 7,42 (je 1H, bs, $CONH_2$); 7,23 (2H, s, $NH_2$); 7,76 und 9,12 (je 1H, s, Im-H); 9,65 (1H, d, J=8Hz, NH) |
| 7 | Cl | $CH_3$ | H | 1,65 - 2,0 (4H, m, 2 x $CH_2$); 2,7 - 2,85 (2H, m, $CH_2$); 3,11 und 3,45 (2H, AB, J=18Hz, $SCH_2$); 3,83 (3H, s, $OCH_3$); 4,10 - 4,25 (2H, m, $CH_2$); 4,98 (1H, d, J=5Hz, 6-H); 5,03 und 5,13 (2H, AB, J=15Hz, $CH_2N^{\oplus}$); 5,61 (1H, dd, J=5 und 8Hz, 7-H); 7,38 (2H, s, $NH_2$); 7,79 und 9,13 (je 1H, s, Im-H); 9,48 (1H, d, J=8Hz, NH); |
| 8 | H | H | H | 1,6 - 1,9 (4H, m, 2 x $CH_2$); 2,7 - 2,8 (2H, m, $CH_2$); 3,2 - 3,4 (2H, bs, $SCH_2$); 4,1 - 4,2 (2H, m, $CH_2$); 4,7 - 4,9 (2H, AB, $CH_2N^{\oplus}$); 5,02 (1H, d, J=5Hz, 6-H); 5,62 (1H, dd, J=5 und 8Hz, 7-H); 7,15 (2H, s, $NH_2$); 7,75 und 8,98 (je 1H, s, Im-H); 9,52 (1H, d, J=8Hz, NH) |
| 9 | H | $CH_2COOH$ | H | (270 MHz):1,7-1,85 (2H, m, $CH_2$); 1,85 - 2,0 (2H, m, $CH_2$); 2,65 - 2,9 (2H, m, $CH_2$); 3,06 und 3,48 (2H, AB, J=18Hz, $SCH_2$); 4,05 - 4,3 (2H, m, $CH_2$); 4,16 (2H, d, J=5Hz, $OCH_2$); 4,60 und 5,69 (2H, |

| Beispiel | $R^1$ | $R^2$ | $R^{3'}$ | $^1H$-NMR(DMSO-$d_6$); 60MHz : δ (ppm)= |
|---|---|---|---|---|
| | | | | AB, J=15Hz, $CH_2N^{\oplus}$); 4,97 (1H, d, J=5Hz, 6-H); 5,67 (1H, dd, J=5 und 8Hz, 7-H); 6,81 (1H, s, Thiazol); 7,14 (2H, s, $NH_2$); 7,79 und 9,18 (je 1H, s, Im-H); 12,10 (1H, d, J=8Hz, NH) |
| 10 | H | $C(CH_3)_2CO_2H$ | H | (270MHz):1,32 und 1,41 (je 3H, s, 2 x $CH_3$); 1,7 - 1,82 (2H, m, $CH_2$); 1,82 - 1,97 (2H, m, $CH_2$); 2,7 - 2,88 (2H, m, $CH_2$); 3,06 und 3,43 (2H, AB, J=18Hz, $SCH_2$); 4,08 - 4,32 (2H, m, $CH_2$); 4,62 und 5,20 (2H, AB, J=15Hz, $CH_2N^{\oplus}$); 4,98 (1H, d, J=5Hz, 6-H), 5,72 (1H, dd, J=5 und 8Hz, 7-H); 6,70 (1H, s, Thiazol); 7,12 (2H, s, $NH_2$); 7,78 und 9,21 (je 1H, s, Im-H); 12,18 (1H, d, J=8Hz, NH) |
| 11 | H | $CH_2-\underset{\underset{CH_2}{\Vert}}{C}CO_2H$ | H | 1,65 - 1,95 (4H, m, 2 x $CH_2$); 2,7 - 2,9 (2H, m, $CH_2$, 3,0- 3,6 (2H, AB, $SCH_2$); 4,1 - 4,2 (2H, m, $CH_2$); 4,71 (2H, s, $OCH_2$); 4,64 und 5,08 (2H, AB, J=15Hz, $CH_2N^{\oplus}$); 5,00 (1H, d, J=5Hz, 6-H), 5,68 (1H, dd, J=5 und 8Hz, 7-H); 5,65 und 6,02 (je 1H, bs, $=CH_2$); 6,72 (1H, s, Thiazol); 7,13 (2H, s, $NH_2$); 7,75 und 9,16 (je 1H, s, Im-H); 11,85 (1H, d, J=8Hz, NH) |

| Beispiel | $R^1$ | $R^2$ | $R^{3'}$ | $^1H$-NMR(DMSO-$d_6$), 60MHz: δ (ppm)= |
|---|---|---|---|---|
| 12 | H | $CH_3$ | 6-$CH_3$ | 1,7 - 2,0 (3H, m, CH und $CH_2$); 2,48 (3H, s, $CH_3$); 2,7 - 2,85 (2H, m, $CH_2$); 3,02 und 3,48 (2H, AB, $SCH_2$); 3,81 (3H, s, $OCH_3$); 4,1 - 4,2 (2H, m, $CH_2$); 4,5 - 5,3 (2H, AB, $CH_2N^+$); 5,01 (1H, d, J=5Hz, 6-H); 5,60 (1H, dd, J=5 und 8Hz), 7-H); 6,72 (1H, s, Thiazol); 7,15 (2H, s, $NH_2$); 7,76 und 9,08 (je 1H, s, Im-H); 9,55 (1H, d, J=8Hz, NH); |

Analog Beispiel 1, Verfahren a) oder b) werden die nachfolgend aufgeführten Beispiele als amorphe Feststoffe erhalten, die der allgemeinen Formel Ib entsprechen und worin R¹, R² und $R^{3'}$ die in der Tabelle 2 angegebene Bedeutung haben.

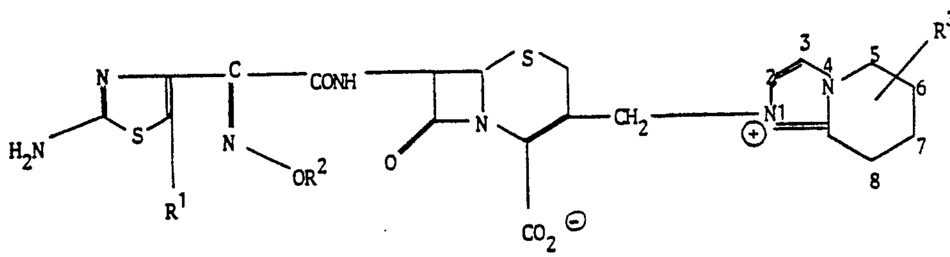

(Ib)

Tabelle 2

| Beipiel | $R^1$ | $R^2$ | $R^{3'}$ | $^1H$-NMR(DMSO-$d_6$), 60MHz : δ (ppm)= |
|---|---|---|---|---|
| 13 | H | $CH_3$ | H | (270MHz): 1,78 - 2,04 (4H, m, 2 x $CH_2$); 2,96 - 3,04 (2H, m, $CH_2$); 3,11 und 3,38 (2H, AB, J=18Hz, $SCH_2$); 3,80 (3H, s, $OCH_3$); 4,02 - 4,12 (2H, m, $CH_2$); 4,84 und 4,92 (2H, AB, J=14Hz, $CH_2N^+$); 5,00 (1H, d, J=5Hz, 6-H); 5,60 (1H, |

| Beispiel | $R^1$ | $R^2$ | $R^{3'}$ | $^1H$-NMR($DMSO-d_6$), 60MHz: $\delta$ (ppm)= |
|---|---|---|---|---|
| | | | | dd, J=5 und 8Hz, 7-H); 6,71 (1H, s, Thiazol); 7,21 (2H, s, $NH_2$); 7,54 und 7,91 (je 1H, d, J=2Hz, Im-H); 9,51 (1H, d, J=8Hz, NH); |
| 14 | Cl | $CH_3$ | H | (270MHz): 1,8 - 2,05 (4H, m, 2 x $CH_2$); 2,95 - 3,1 (2H, m, $CH_2$); 3,0 - 3,4 (2H, AB, $SCH_2$); 3,81 (3H, s, $OCH_3$); 4,0 - 4,15 (2H, m, $CH_2$); 4,75 - 5,0 (2H, AB, $CH_2N^{\oplus}$); 4,96 (1H, d, J=5Hz, 6-H); 5,58 (1H, dd, J=5 und 8Hz, 7-H); 7,38 (2H, s, $NH_2$); 7,52 und 7,89 (je 1H, d, J=2Hz, Im-H); 9,45 (1H, d, J=8Hz, NH) |
| 15 | H | $C_2H_5$ | H | (270MHz): 1,21 (3H, t, $CH_2\underline{CH_3}$); 1,8 - 2,05 (4H, m, 2 x $CH_2$); 2,95 - 3,05 (2H, m, $CH_2$); 3,11 und 3,32 (2H, AB, J=18Hz, $SCH_2$); 4,08 (2H, q, $\underline{CH_2}CH_3$); 4,0 - 4,15 (2H, m, $CH_2$); 4,84 und 4,92 (2H, AB, J=14Hz, $CH_2N^{\oplus}$); 5,01 (1H, d, J=5Hz, 6-H); 5,61 (1H, dd, J=5 und 8Hz, 7-H); 6,69 (1H, s, Thiazol); 7,20 (2H, s, $NH_2$); 7,54 und 7,91 (je 1H, d, J=2Hz, Im-H); 9,46 (1H, d, J=8Hz, NH) |
| 16 | H | $CH_2CONH_2$ | H | (270MHz): 1,8- 2,05 (4H, m, 2 x $CH_2$); 2,95 - 3,05 (2H, m, $CH_2$); 3,21 und 3,48 (2H, AB, J=18Hz, $SCH_2$); 4,05 - 4,15 (2H, m, $CH_2$); 4,38 (2H, s, $OCH_2$); 4,90 und 4,95 |

| Beispiel | $R^1$ | $R^2$ | $R^{3'}$ | $^1H$-NMR(DMSO-$d_6$), 60MHz : δ (ppm)= |
|---|---|---|---|---|
| | | | | (2H, AB, J=14Hz, $CH_2N^{\oplus}$); 5,08 (1H, d, J=5Hz, 6-H); 5,72 (1H, dd, J=5 und 8Hz, 7-H); 6,82 (1H, s, Thiazol); 7,09 und 7,45 (je 1H, bs, $CONH_2$); 7,26 (2H, bs, $NH_2$); 7,56 und 7,81 (je 1H, d, J=2Hz, Im-H); 9,72 (1H, d, J=8Hz, NH) |
| 17 | H | $CH_2COOH$ | H | (270MHz): 1,85 - 2,05 (4H, m, 2 x $CH_2$); 2,95 - 3,05 (2H, m, $CH_2$); 3,07 und 3,32 (2H, AB, J=18Hz, $SCH_2$); 4,02 - 4,12 (2H, m, $CH_2$); 4,13 (2H, d, J=5Hz, $OCH_2$); 4,80 und 4,98 (2H, AB, J=15Hz, $CH_2N^{\oplus}$); 4,98 (1H, d, J=5Hz, 6-H); 5,62 (1H, dd, J=5 und 8Hz, 7-H); 6,82 (1H, s, Thiazol); 7,15 (2H, s, $NH_2$); 7,54 undd 8,02 (je 1H, s, Im-H); 11,25 (1H, d, J=8Hz, NH); |
| 18 | H | $C(CH_3)_2CO_2H$ | H | (270MHz): 1,34 und 1,43 (je 3H, s, 2 x $CH_3$); 1,8 - 2,05 (4H, m, 2 x $CH_2$); 2,95 - 3,08 (2H, m, $CH_2$); 3,08 und 3,38 (2H, AB, J=18Hz, $SCH_2$); 4,0 - 4,15 (2H, m, $CH_2$); 4,88 und 4,95 (2H, AB, J=15Hz, $CH_2N^{\oplus}$); 4,98 (1H, d, J=5Hz, 6-H); 5,68 (1H, dd, J=5 und 8Hz, 7-H); 6,72 (1H, s, Thiazol); 7,15 (2H, s, $NH_2$); 7,55 und 7,94 (je 1H, s, Im-H); 11,18 (1H, d, J=8Hz, NH |

| Beispiel | $R^1$ | $R^2$ | $R^{3'}$ | $^1$H-NMR(DMSO-$d_6$): 60MHz, δ (ppm)= |
|---|---|---|---|---|
| 19 | H | $CH_3$ | 2-$CH_3$ | 1,8 - 2,1 (4H, m, 2 x $CH_2$); 2,35 (3H, s, $CH_3$); 2,9 - 3,1 (2H, m, $CH_2$); 3,0 - 3,5 (2H, AB, $SCH_3$); 3,81 (3H, s, $OCH_3$); 4,0 - 4,2 (2H, m, $CH_2$); 4,6 - 5,1 (2H, AB, $CH_2N^{\oplus}$); 5,01 (1H, d, J=5Hz, 6-H); 5,62 (1H, dd, J=5 und 8Hz, 7-H); 6,70 (1H, s, Thiazol); 7,21 (2H, s, $NH_2$); 7,68 (1H, s, Im-H); 9,52 (1H, d, J=8Hz, NH); |
| 20 | H | $CH_3$ | 8-OH | 1,7 - 2,0 (4H, m, 2 x $CH_2$); 3,0 - 3,6 (2H, AB, $SCH_2$); 3,80 (3H, s, $OCH_3$); 3,9 - 4,1 (2H, m, $CH_2$); 4,6 - 5,2 (3H, m, $CH_2N^{\oplus}$ und CHOH); 5,02 (1H, d, J=5Hz, 6-H); 5,60 (1H, dd, J=5 und 8Hz, 7-H); 6,71 (1H, s, Thiazol); 7,25 (2H, s, $NH_2$); 7,55 und 7,87 (je 1H, d, J=2Hz, Im-H); 9,48 (1H, d, J=8Hz, NH); |
| 21 | H | $CH_3$ | 2-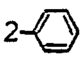 | 1,7 - 2,1 (4H, m, 2 x $CH_2$); 2,9 - 3,2 (2H, m, $CH_2$); 3,1 - 3,5 (2H, AB, $SCH_2$); 3,81 (3H, s, $OCH_3$); 4,0 - 4,2 (2H, m, $CH_2$); 4,6 - 5,1 (2H, AB, $CH_2N^{\oplus}$); 5,03 (1H, d, J=5Hz, 6-H); 5,61 (1H, dd, J=5 und 8Hz, 7-H); 6,70 (1H, s, Thiazol); 6,90 - 8,0 (8H, m, $NH_2$, 5 Phenyl-H, 1 Im-H); 9,50 (1H, d, J=8Hz, NH); |

| Beispiel | $R^1$ | $R^2$ | $R^{3'}$ | $^1H$-NMR(DMSO-$d_6$) 60MHz, δ (ppm)= |
|---|---|---|---|---|
| 22 | H | $CH_3$ | 2-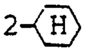 | 1,0 - 2,1 (14H, m, 10 Cyclohexyl-H, 2 x $CH_2$); 2,6 - 3,1 (3H, m, $CH_2$, 1 Cyclohexyl-H); 3,0 - 3,5 (2H, AB, $SCH_2$); 3,82 (3H, s, $OCH_3$); 4,0 - 4,2 (2H, m, $CH_2$); 4,88 (2H, m, $CH_2N^{\oplus}$); 5,00 (1H, d, J=5Hz, 6-H); 5,58 (1H, dd, J=5 und 8Hz, 7-H); 6,70 (1H, s, Thiazol); 7,25 (2H, s, $NH_2$); 7,65 (1H, s, Im-H); 9,48 (1H, d, J=8Hz, NH) |
| 23 | H | $CH_3$ | 3-$CONH_2$ | 1,7 - 2,1 (4H, m, 2 x $CH_2$); 2,9 - 3,1 (2H, m, $CH_2$); 3,1 - 3,5 (2H, AB, $SCH_2$); 3,80 (3H, s, $OCH_3$); 4,0 - 4,2 (2H, m, $CH_2$); 4,6 - 4,9 (2H, AB, $CH_2N^{\oplus}$); 5,00 (1H, d, J=5Hz, 6-H); 5,59 (1H, dd, J=5 und 8Hz, 7-H); 6,70 (1H, s, Thiazol); 6,9 - 7,4 (4H, 2 x $NH_2$); 7,78 (1H, s, Im-H); 9,50 (1H, d, J=8Hz, NH); |
| 24 | H | $CH_3$ | 3-$CO_2CH_3$ | 1,7 - 2,0 (4H, m, 2 x $CH_2$); 2,9 - 3,1 (2H, m, $CH_2$); 3,0 - 3,5 (2H, AB, $SCH_2$); 3,73 und 3,80 (je 3H, s, 2x$OCH_3$); 4,0 - 4,2 (2H, m, $CH_2$); 4,7 - 5,2 (2H, AB, $CH_2N^{\oplus}$); 5,01 (1H, d, J=5Hz, 6-H); 5,60 (1H, dd, J=5 und 8Hz, 7-H); 6,70 (1H, s, Thiazol); 7,20 (2H, s, $NH_2$); 7,72 (1H, s, Im-H); 9,55 (1H, d, J=8Hz, NH) |

| Beispiel | $R^1$ | $R^2$ | $R^{3'}$ | $^1H$-NMR(DMSO-$d_6$)60MHz; $\delta$(ppm)= |
|---|---|---|---|---|
| 25 | H | $CH_3$ | 3-COOH | 1,8 - 2,1 (4H, m, 2 x $CH_2$); 2,9 - 3,1 (2H, m, $CH_2$); 3,03 - 3,6 (2H, AB, $SCH_2$); 3,81 (3H, s, $OCH_3$); 4,0 - 4,15 (2H, m, $CH_2$); 4,5 - 5,0 (2H, AB, $CH_2N^{\oplus}$); 5,02 (1H, d, J=5Hz, 6-H); 5,60 (1H, dd, J=5 und 8Hz, 7-H); 6,72 (1H, s, Thiazol), 7,28 (2H, s, $NH_2$); 7,82 (1H, s, Im-H); 9,68 (1H, d, J=8Hz, NH); |
| 26 | H | $CH_3$ | 8-$OCH_3$ | 1,8 - 2,0 (4H, m, 2 x $CH_2$); 3,1 - 3,5 (2H, AB, $SCH_2$); 3,75 und 3,82 (je 3H, s, 2 x $OCH_3$); 3,9 - 4,1 (2H, m, $CH_2$); 4,5 - 5,1 (3H, m, $CH_2N^{\oplus}$ und <u>CH</u>$OCH_3$, 5,00 (1H, d, J=5Hz, 6-H); 5,61 (1H, dd, J=5 und 8Hz, 7-H); 6,70 (1H, s, Thiazol); 7,22 (2H, s, $NH_2$); 7,53 und 7,90 (je 1H, d, J=2Hz, Im-H); 9,52 (1H, d, J=8Hz, NH); |

**Ansprüche**

1. Cephalosporinderivate der allgemeinen Formel I

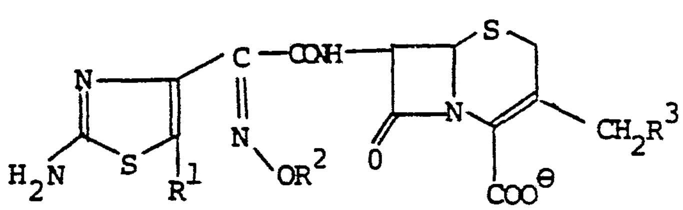

( I )

und deren physiologisch verträgliche Säureadditionssalze worin die $R^2O$-Gruppe in syn-Stellung steht und die Substituenten $R^1$, $R^2$ und $R^3$ die folgenden Bedeutungen besitzen:
$R^1$ Wasserstoff oder Halogen
$R^2$ Wasserstoff,
$C_1$-$C_6$-Alkyl, das ein-oder mehrfach, gleich oder verschieden substituiert sein kann durch Halogen, Aryl,

Heteroaryl, $C_1$-$C_4$-Alkylthio, $C_1$-$C_4$-Alkoxy, Nitril oder Carbamoyl, worin die Aminogruppe ein-oder zweifach substituiert sein kann durch $C_1$-$C_4$-Alkyl,

$C_2$-$C_6$-Alkenyl, das gegebenenfalls ein-oder mehrfach substituiert sein kann durch Halogen.

$C_2$-$C_6$-Alkinyl,

$C_3$-$C_7$-Cycloalkyl,

$C_4$-$C_7$-Cycloalkenyl,

$C_3$-$C_7$-Cycloalkylmethyl,

die Gruppe

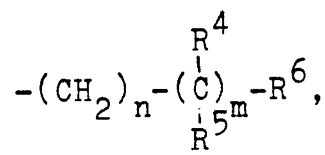

worin m und n jeweils für 0 oder 1 steht und $R^4$ und $R^5$ gleich oder verschieden sein können und Wasserstoff oder eine $C_1$-$C_{11}$-Alkylgruppe bedeuten, oder zusammen mit dem Kohlenstoff, an das sie gebunden sind, eine Vinyliden-oder eine $C_3$-$C_7$-Cycloalkylidengruppe bilden,

$R^6$ eine Carboxy-oder $C_1$-$C_4$Alkoxycarbonylgruppe,

$R^3$ einen 5,6,7,8-Tetrahydroimidazo[1,2-a]pyridiniumrest (a) oder einen 5,6,7,8-Tetrahydroimidazo[1,5-a]-pyridiniumrest (b)

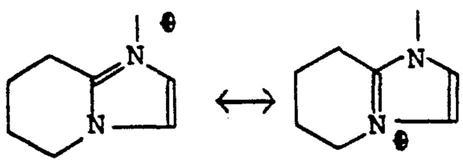
(a)

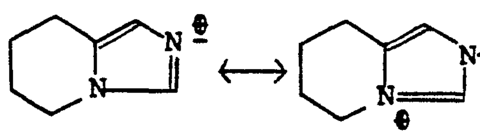
(b)

der ein-oder mehrfach, gleich oder verschieden substituiert sein kann durch $C_1$-$C_6$-Alkyl, das auch noch durch Hydroxy, $C_1$-$C_6$-Alkyloxy, Carboxy, $C_1$-$C_6$-Alkoxycarbonyl, Carbamoyl oder Amino substituiert sein kann;

$C_2$-$C_6$-Alkenyl, $C_3$-$C_6$Alkinyl, $C_3$-$C_6$-Cycloalkyl, $C_5$-$C_6$-Cycloalkenyl, $C_3$-$C_6$-Cycloalkylmethyl; Phenyl, Phenoxy oder Benzyl, die durch $C_1$-$C_4$-Alkyl, Hydroxy, $C_1$-$C_4$-Alkoxy substituiert sein können;

$C_1$-$C_6$-Alkoxy, $C_1$-$C_6$-Alkylthio, Sulfo, Trifluormethyl, Hydroxy, Mercapto, Amino, $C_1$-$C_6$-Alkylamino, Di-$C_1$-$C_6$-Alkylamino, Carboxy, $C_1$-$C_6$-Alkoxycarbonyl oder Carbamoyl, das am Stickstoff ein-oder zweimal substituiert sein kann durch $C_1$-$C_6$-Alkyl.

2. Verfahren zur Herstellung der Cephalosporinderivate der allgemeinen Formel I und ihrer physiologisch verträglichen Säureadditionssalze, dadurch gekennzeichnet, daß man

a) eine Verbindung der allgemeinen Formel II

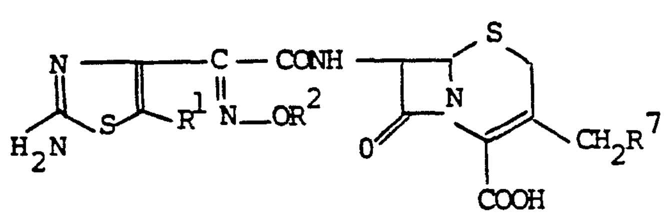

oder deren Salze, worin $R^1$ und $R^2$ die in Formel I genannte Bedeutung haben, die Aminogruppe auch ge-

schützt sein kann, und $R^7$ eine durch Tetrahydroimidazopyridin oder dasjenige Tetrahydroimidazopyridin-Derivat, das dem Rest $R^3$ der Formel I entspricht, austauschbare Gruppe bedeutet, mit Tetrahydroimidazo-pyridin oder diesem Tetrahydroimidazo-pyridin-Derivat umsetzt,

α) eine gegebenenfalls vorhandene Schutzgruppe abspaltet und

β) falls erforerlich, das erhaltene Produkt in ein physiologisch verträgliches Säureadditionssalz überführt,

oder

b) eine Verbindung der allgemeinen Formel III

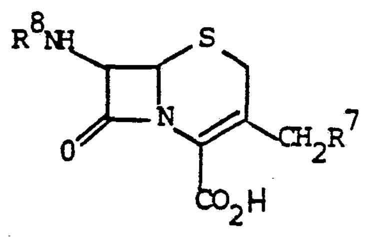

(III)

worin $R^7$ die vorstehend für die Formel II genannte Bedeutung hat und $R^8$ für Wasserstoff oder eine Aminoschutzgruppe steht, mit Tetrahydroimidazopyridin oder dem Tetrahydroimidazopyridin-Derivat, das dem in Formel I definierten Rest $R^3$ zugrunde liegt, umsetzt unter Bildung der Verbindung der allgemeinen Formel IV,

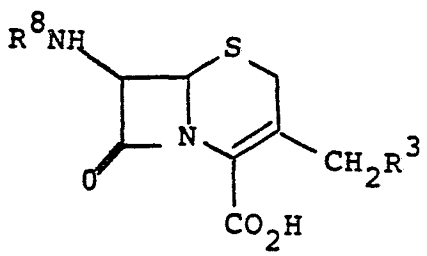

(IV)

in der $R^3$ und $R^8$ die oben genannte Bedeutung haben und

α) eine gegebenenfalls vorhandene Aminoschutzgruppe abspaltet und

β) die Verbindung IV, worin $R^8$ Wasserstoff bedeutet, entweder als solche oder in Form eines reaktionsfähigen Derivates mit einer 2-syn-Oxyiminoessigsäure der allgemeinen Formel V,

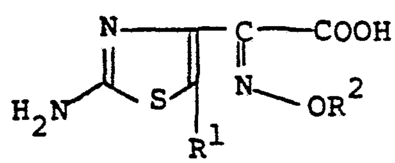

(V)

worin $R^1$ und $R^2$ die genannte Bedeutung besitzen und die Aminogruppe auch in geschützter Form vorliegen kann, oder mit einem an der Carboxylgruppe aktivierten Derivat dieser Verbindung umsetzt und

α) eine gegebenenfalls vorhandene Schutzgruppe abspaltet und

β) falls erforderlich, das erhaltene Produkt der allgemeinen Formel I in ein physiologisch verträgliches Säureadditionssalz überführt.

3. Gegen bakterielle Infektionen wirksame pharamzeutische Präparate, gekennzeichnet durch einen Gehalt an Cephalosporinderivaten der allgemeinen Formel I.

4. Verfahren zur Herstellung von gegen bakterielle Infektionen wirksamen pharmazeutischen Präparaten, dadurch gekennzeichnet, daß ein Cephalosporinderivat der allgemeinen Formel I gegebenenfalls mit pharmazeutisch üblichen Trägerstoffen oder Verdünnungsmitteln in eine pharmazeutisch geeignete Verabreichungsform gebracht wird.

5. Verwendung von Cephalosporinderivaten der allgemeinen Formel I zur Bekämpfung bakterieller Infektionen.

Patentansprüche für die folgenden Vertragsstaaten: ES, GR

1. Verfahren zur Herstellung von Cephalosporinderivaten der allgemeinen Formel I

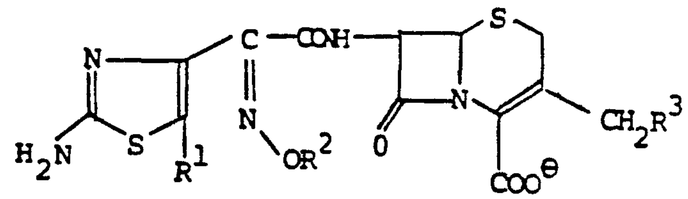

( I )

und deren physiologisch verträglichen Säureadditionssalzen worin die $R^2O$-Gruppe in syn-Stellung steht und die Substituenten $R^1$, $R^2$ und $R^3$ die folgenden Bedeutungen besitzen:

$R^1$ Wasserstoff oder Halogen

$R^2$ Wasserstoff,

$C_1$-$C_6$-Alkyl, das ein-oder mehrfach, gleich oder verschieden substituiert sein kann durch Halogen, Aryl, Heteroaryl, $C_1$-$C_4$-Alkylthio, $C_1$-$C_4$-Alkoxy, Nitril oder Carbamoyl, worin die Aminogruppe ein-oder zweifach substituiert sein kann durch $C_1$-$C_4$-Alkyl,

$C_2$-$C_6$-Alkenyl, das gegebenenfalls ein-oder mehrfach substituiert sein kann durch Halogen,

$C_2$-$C_6$-Alkinyl,

$C_3$-$C_7$-Cycloalkyl,

$C_4$-$C_7$-Cycloalkenyl,

$C_3$-$C_7$-Cycloalkylmethyl,

die Gruppe

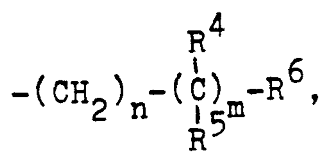

worin m und n jeweils für 0 oder 1 steht und $R^4$ und $R^5$ gleich oder verschieden sein können und Wasserstoff oder eine $C_1$-$C_{11}$-Alkylgruppe bedeuten, oder zusammen mit dem Kohlenstoff, an das sie gebunden sind, eine Vinyliden-oder eine $C_3$-$C_7$-Cycloalkylidengruppe bilden,

$R^6$ eine Carboxy-oder $C_1$-$C_4$Alkyloxycarbonylgruppe,

$R^3$ einen 5,6,7,8-Tetrahydroimidazo[1,2-a]pyridiniumrest (a) oder einen 5,6,7,8-Tetrahydroimidazo[1,5-a]-pyridiniumrest (b)

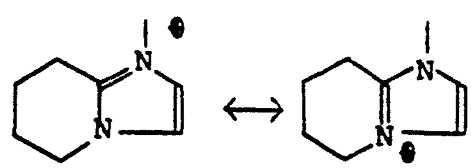

(a)

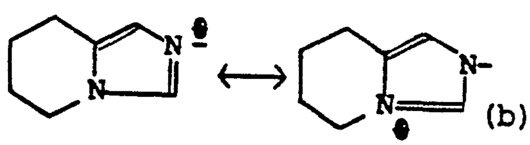

(b)

der ein-oder mehrfach, gleich oder verschieden substituiert sein kann durch $C_1$-$C_6$-Alkyl, das auch noch durch Hydroxy, $C_1$-$C_6$-Alkyloxy, Carboxy, $C_1$-$C_6$-Alkoxycarbonyl, Carbamoyl oder Amino substituiert sein kann;

$C_2$-$C_6$-Alkenyl, $C_3$-$C_6$Alkinyl, $C_3$-$C_6$-Cycloalkyl, $C_5$-$C_6$-Cycloalkenyl, $C_3$-$C_6$-Cycloalkylmethyl; Phenyl, Phenoxy

oder Benzyl, die durch $C_1$-$C_4$-Alkyl, Hydroxy, $C_1$-$C_4$-Alkoxy substituiert sein können; $C_1$-$C_6$-Alkoxy, $C_1$-$C_6$-Alkylthio, Sulfo, Trifluormethyl, Hydroxy, Mercapto, Amino, $C_1$-$C_6$-Alkylamino, Di-$C_1$-$C_6$-Alkylamino, Carboxy, $C_1$-$C_6$-Alkoxycarbonyl oder Carbamoyl, das am Stickstoff ein-oder zweimal substituiert sein kann durch $C_1$-$C_6$-Alkyl,

dadurch gekennzeichnet, daß man

a) eine Verbindung der allgemeinen Formel II

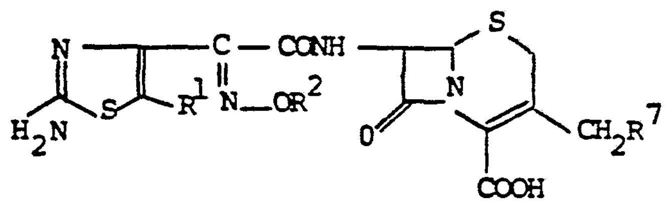

(II)

oder deren Salze, worin $R^1$ und $R^2$ die in Formel I genannte Bedeutung haben, die Aminogruppe auch geschützt sein kann, und $R^7$ eine durch Tetrahydroimidazopyridin oder dasjenige Tetrahydroimidazopyridin-Derivat, das dem Rest $R^3$ der Formel I entspricht, austauschbare Gruppe bedeutet, mit Tetrahydroimidazopyridin oder diesem Tetrahydroimidazo-pyridin-Derivat umsetzt,

α) eine gegebenenfalls vorhandene Schutzgruppe abspaltet und

β) falls erforerlich, das erhaltene Produkt in ein physiologisch verträgliches Säureadditionssalz überführt,

oder

b) eine Verbindung der allgemeinen Formel III

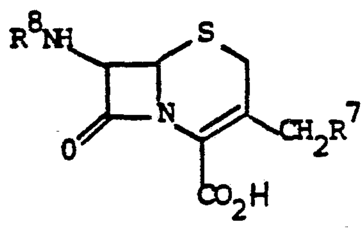

(III)

worin $R^7$ die vorstehend für die Formel II genannte Bedeutung hat und $R^8$ für Wasserstoff oder eine Aminoschutzgruppe steht, mit Tetrahydroimidazopyridin oder dem Tetrahydroimidazopyridin-Derivat, das dem in Formel I definierten Rest $R^3$ zugrunde liegt, umsetzt unter Bildung der Verbindung der allgemeinen Formel IV,

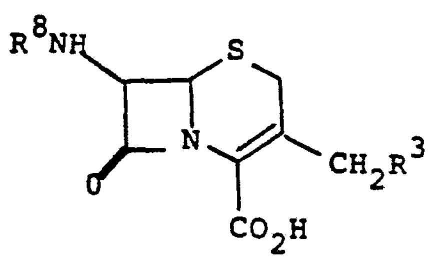

(IV)

in der $R^3$ und $R^8$ die oben genannte Bedeutung haben und

α) eine gegebenenfalls vorhandene Aminoschutzgruppe abspaltet und

β) die Verbindung IV, worin $R^8$ Wasserstoff bedeutet, entweder als solche oder in Form eines reaktionsfähigen Derivates mit einer 2-syn-Oxyiminoessigsäure der allgemeinen Formel V,

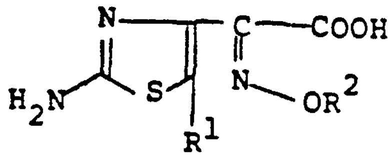

(V)

worin $R^1$ und $R^2$ die genannte Bedeutung besitzen und die Aminogruppe auch in geschützter Form vorliegen kann, oder mit einem an der Carboxylgruppe aktivierten Derivat dieser Verbindung umsetzt und

α) eine gegebenenfalls vorhandene Schutzgruppe abspaltet und

β) falls erforderlich, das erhaltene Produkt der allgemeinen Formel I in ein physiologisch verträgliches Säureadditionssalz überführt.

2. Verfahren zur Herstellung von gegen bakterielle Infektionen wirksamen pharmazeutischen Präparaten, dadurch gekennzeichnet, daß ein Cephalosporinderivat der allgemeinen Formel I gegebenenfalls mit pharmazeutisch üblichen Trägerstoffen oder Verdünnungsmitteln in eine pharmazeutisch geeignete Verabreichtungsform gebracht wird.